(19) **Europäisches Patentamt / European Patent Office / Office européen des brevets**

(11) **EP 4 270 401 A1**

(12) **EUROPEAN PATENT APPLICATION**
published in accordance with Art. 153(4) EPC

(43) Date of publication:
**01.11.2023 Bulletin 2023/44**

(21) Application number: **21910121.9**

(22) Date of filing: **25.11.2021**

(51) International Patent Classification (IPC):
**G16C 20/64** (2019.01)     **C07B 61/00** (2006.01)
**G06F 16/9038** (2019.01)

(52) Cooperative Patent Classification (CPC):
**C07B 61/00; G06F 16/9038; G16C 20/64**

(86) International application number:
**PCT/JP2021/043214**

(87) International publication number:
**WO 2022/137968 (30.06.2022 Gazette 2022/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **25.12.2020 JP 2020216936**

(71) Applicant: **FUJIFILM Corporation**
**Tokyo 106-8620 (JP)**

(72) Inventors:
• **YARIMIZU, Hirokazu**
**Tokyo 106-8620 (JP)**
• **HIKIDA, Yasushi**
**Tokyo 106-8620 (JP)**

(74) Representative: **Meissner Bolte Partnerschaft mbB**
**Widenmayerstrasse 47**
**80538 München (DE)**

(54) **INFORMATION PROCESSING DEVICE, INFORMATION PROCESSING METHOD, AND INFORMATION PROCESSING PROGRAM**

(57)     An information processing apparatus performs a first search for searching structure data indicating a structure of a chemical substance, which is a processing target, for a partial structure included in partial structure data in which the partial structure of the chemical substance and an index value indicating performance or a structure of the partial structure are associated with each other, performs a second search for searching past data in which the structure of the chemical substance and an index value indicating performance or the structure of the chemical substance obtained by an experiment are associated with each other for a chemical substance including the partial structure extracted by the first search, derives a reliability degree of the index value of the partial structure based on a total number of the chemical substances extracted by the second search and an index value corresponding to the chemical substance, and performs control of displaying the partial structure extracted by the first search and the reliability degree of the partial structure on a display device.

FIG. 7

EP 4 270 401 A1

**Description**

**BACKGROUND OF THE INVENTION**

1. Field of the Invention

The present disclosure relates to an information processing apparatus, an information processing method, and an information processing program.

2. Description of the Related Art

JPWO2009/118845A1 discloses that index values such as the presence or absence of the carcinogenicity and the toxicity of each of a prediction target compound and a virtual compound generated based on the prediction target compound are predicted, a reliability degree of the prediction value of the prediction target compound is obtained from each prediction result, and the reliability degree is displayed.

JP2015-052990A discloses that an overall structure diagram is searched from a structural formula database based on a compound name formula, the compound name is decomposed into partial structure names, and a structure index that is likely to be useful for the association with the overall structure among the partial structures obtained by the decomposition is presented.

**SUMMARY OF THE INVENTION**

In the field of chemistry, in a case in which a chemical substance is developed, for example, screening is performed based on index values such as the presence or absence of carcinogenicity and toxicity. In the screening, the presence or absence of a partial structure (hereinafter, referred to as a "concern structure") in which there is a concern of adverse influence on a predetermined index value is taken into consideration.

However, the concern structure is generally defined by the experience and knowledge of an expert, and the reliability thereof may be low. Even in a case in which the screening is performed based on such a partial structure, the screening may not be performed appropriately. In the technologies disclosed in JPWO2009/118845A1 and JP2015-052990A, the reliability of the partial structure is not taken into consideration.

The present disclosure has been made in view of the above circumstances, and the present disclosure is to provide an information processing apparatus, an information processing method, and an information processing program capable of supporting appropriate screening.

The present disclosure relates to an information processing apparatus comprising at least one processor, in which the processor performs a first search for searching structure data indicating a structure of a chemical substance, which is a processing target, for a partial structure included in partial structure data in which the partial structure of the chemical substance and an index value indicating performance or a structure of the partial structure are associated with each other, performs a second search for searching past data in which the structure of the chemical substance and an index value indicating performance or the structure of the chemical substance obtained by an experiment are associated with each other for a chemical substance including the partial structure extracted by the first search, derives a reliability degree indicating reliability of the index value of the partial structure extracted by the first search based on a total number of the chemical substances extracted by the second search and an index value corresponding to the chemical substance, and performs control of displaying the partial structure extracted by the first search and the reliability degree of the partial structure on a display device.

It should be noted that, in the information processing apparatus according to the present disclosure, the processor may perform control of displaying the reliability degree on the partial structure of the chemical substance, which is the processing target, in a discriminable manner.

In addition, in the information processing apparatus according to the present disclosure, the processor may perform control of displaying the reliability degree in descending order of the reliability degree in a case in which there are a plurality of the partial structures extracted by the first search.

In addition, in the information processing apparatus according to the present disclosure, the processor may perform weighting according to the total number of the chemical substances extracted by the second search in a case in which the reliability degree is derived.

In addition, the present disclosure relates to an information processing method executed by a processor provided in an information processing apparatus, the method comprising performing a first search for searching structure data indicating a structure of a chemical substance, which is a processing target, for a partial structure included in partial structure data in which the partial structure of the chemical substance and an index value indicating performance or a structure of the partial structure are associated with each other, performing a second search for searching past data in which the structure of the chemical substance and an index value indicating performance or the structure of the chemical substance obtained by an experiment are associated with each other for a chemical substance including the partial structure extracted by the first search, deriving a reliability degree indicating reliability of the index value of the partial structure extracted by the first search based on a total number of the chemical substances extracted by the second search and an index value corresponding to the chemical substance, and performing control of displaying the partial structure extracted by the first search and the reliability degree of the partial structure on a display device.

In addition, the present disclosure relates to an information processing program causing a processor provided in an information processing apparatus to execute a process comprising performing a first search for searching structure data indicating a structure of a chemical substance, which is a processing target, for a partial structure included in partial structure data in which the partial structure of the chemical substance and an index value indicating performance or a structure of the partial structure are associated with each other, performing a second search for searching past data in which the structure of the chemical substance and an index value indicating performance or the structure of the chemical substance obtained by an experiment are associated with each other for a chemical substance including the partial structure extracted by the first search, deriving a reliability degree indicating reliability of the index value of the partial structure extracted by the first search based on a total number of the chemical substances extracted by the second search and an index value corresponding to the chemical substance, and performing control of displaying the partial structure extracted by the first search and the reliability degree of the partial structure on a display device.

According to the present disclosure, it is possible to support appropriate screening.

## BRIEF DESCRIPTION OF THE DRAWINGS

Fig. 1 is a block diagram showing an example of a hardware configuration of an information processing apparatus.
Fig. 2 is a diagram showing a graph showing a structure of a chemical substance.
Fig. 3 is a diagram for describing partial structure data.
Fig. 4 is a diagram showing an example of the partial structure data.
Fig. 5 is a diagram showing an example of past data.
Fig. 6 is a block diagram showing an example of a functional configuration of the information processing apparatus.
Fig. 7 is a diagram showing an example of a reliability degree display screen.
Fig. 8 is a flowchart showing an example of reliability degree display processing.

## DESCRIPTION OF THE PREFERRED EMBODIMENTS

Hereinafter, with reference to the drawings, an embodiment for performing the technology of the present disclosure will be described in detail.

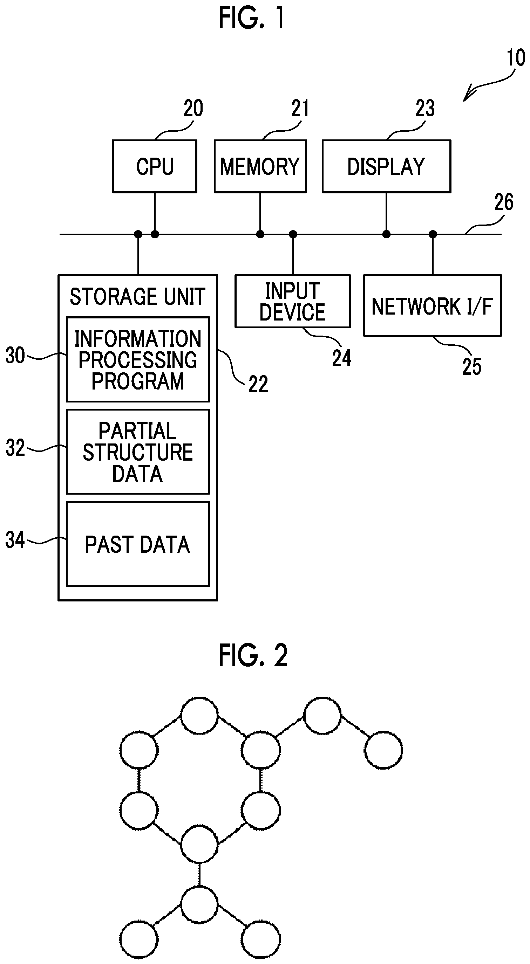

First, with reference to Fig. 1, a hardware configuration of an information processing apparatus 10 according to the present embodiment will be described. As shown in Fig. 1, the information processing apparatus 10 includes a central processing unit (CPU) 20, a memory 21 as a transitory storage area, and a non-volatile storage unit 22. Moreover, the information processing apparatus 10 includes a display 23, such as a liquid crystal display, an input device 24, such as a keyboard and a mouse, and a network interface (I/F) 25 connected to a network. The CPU 20, the memory 21, the storage unit 22, the display 23, the input device 24, and the network I/F 25 are connected to a bus 26. Examples of the information processing apparatus 10 include a personal computer and a server computer.

The storage unit 22 is realized by a hard disk drive (HDD), a solid state drive (SSD), a flash memory, or the like. An information processing program 30 is stored in the storage unit 22 as a storage medium. The CPU 20 reads out the information processing program 30 from the storage unit 22, develops the read out information processing program 30 in the memory 21, and executes the developed information processing program 30.

In addition, partial structure data 32 and past data 34 are stored in the storage unit 22. With reference to Figs. 2 to 5, the partial structure data 32 and the past data 34 will be described.

As shown in Fig. 2 as an example, the information processing apparatus 10 according to the present embodiment handles structure data in which a structure of a chemical substance is represented in a graph format as structure data indicating the structure of the chemical substance. In the structure data, atoms are represented as nodes and bonds are represented as edges. It should be noted that the format of the structure data is not limited to the graph format. For example, as the format of the structure data, a character string format such as a deoxyribonucleic acid (DNA) base sequence may be applied.

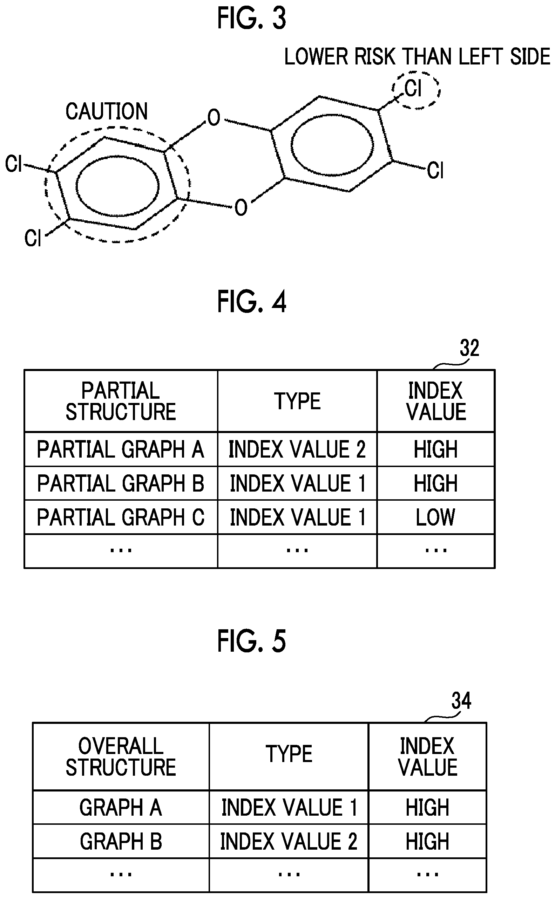

In addition, as shown in Fig. 3 as an example, a part of the structure constituting the chemical substance may influence an index value indicating the performance or the structure of the chemical substance. In the following description, a part of the structure constituting the chemical substance will be referred to as a "partial structure". Examples of the index value indicating the performance of the chemical substance include the presence or absence of carcinogenicity, the presence or absence of toxicity, and a degree of solubility in water. In addition, examples of the index value indicating the structure of the chemical substance include a molecular weight and the number of ring structures. In the following description, in a case in which the index value indicating the performance of the chemical substance and the index value indicating the structure are generically referred to, both index values are simply referred to as an "index value".

Fig. 4 shows an example of the partial structure data 32. As shown in Fig. 4, in the partial structure data 32, the partial structure of the chemical substance is associated with a type of the index value of the partial structure and the index value. In addition, the partial structure data 32 includes a plurality of combinations of the partial structure, and the type of the index value of the partial structure and the index value. A partial graph in Fig. 4 means the structure data in which the partial structure is represented in a graph format. For example, the partial structure data 32 is created in advance based on the experience and knowledge of an expert. It should be noted that, in the partial structure data 32, one partial structure may be associated with one type of index value, or may be associated with a plurality of types of index values.

Fig. 5 shows an example of the past data 34. As shown in Fig. 5, in the past data 34, the structure of the chemical substance is associated with the type of the index value of the chemical substance and the index value, which are obtained by the experiment. In addition, the past data 34 includes a plurality of combinations of the structure of the chemical substance, and the type of the index value of the chemical substance and the index value. The graph in Fig. 5 means the structure data in which an overall structure of the chemical substance is represented in a graph format. The past data 34 is created in advance based on an actual measurement value obtained in the past experiment. It should be noted that, in the past data 34, the structure of one chemical substance may be associated with one type of index value, or may be associated with a plurality of types of index values.

As described above, since the past data 34 is the actual measurement value obtained by the past experiment, the reliability of the index value included in the past data 34 is relatively high. On the other hand, since the index value included in the partial structure data 32 is based on the experience and knowledge of the expert, the reliability may be relatively low. Therefore, the information processing apparatus 10 according to the present embodiment has a function of deriving a reliability degree of the index value of the partial structure constituting the chemical substance, which is the processing target, by using the partial structure data 32 and the past data 34.

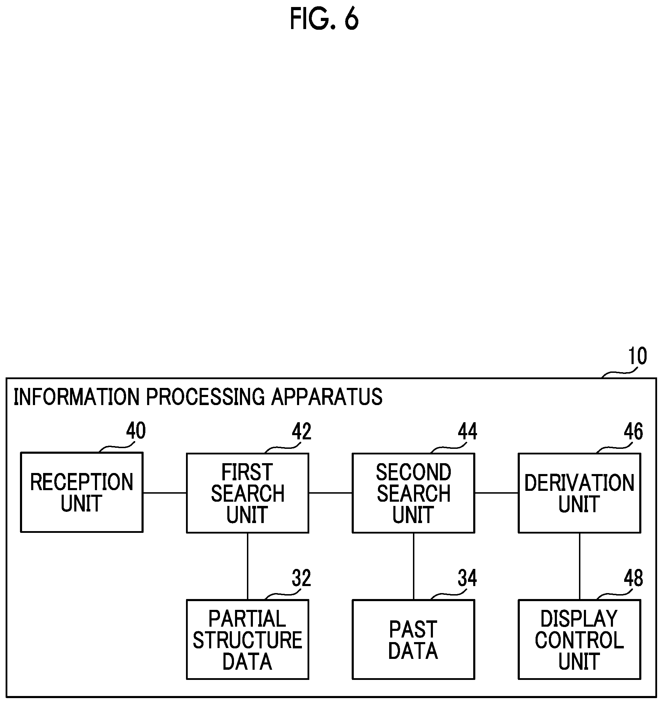

Next, with reference to Fig. 6, a functional configuration of the information processing apparatus 10 according to the present embodiment will be described. As shown in Fig. 6, the information processing apparatus 10 includes a reception unit 40, a first search unit 42, a second search unit 44, a derivation unit 46, and a display control unit 48. The CPU 20 executes the information processing program 30 to function as the reception unit 40, the first search unit 42, the second search unit 44, the derivation unit 46, and the display control unit 48.

The reception unit 40 receives the structure data indicating the structure of the chemical substance, which is the processing target and is input by a user. The user inputs the structure data indicating the structure of the chemical substance by using, for example, a molecular design editor, such as ChemDraw (registered trademark).

The first search unit 42 performs a first search for searching the structure data received by the reception unit 40 for the partial structure included in the partial structure data 32. The second search unit 44 performs a second search for searching the past data 34 for the chemical substance including the partial structure extracted by the first search by the first search unit 42.

The derivation unit 46 derives the reliability degree indicating the reliability of the index value of the partial structure extracted by the first search by the first search unit 42 based on a total number of the chemical substances extracted by the second search by the second search unit 44 and the index values corresponding to the chemical substances. In a case in which the reliability degree is derived, the derivation unit 46 performs weighting according to the total number of the chemical substances extracted by the second search by the second search unit 44. Specifically, the derivation unit 46 derives the reliability degree according to Expression (1).

$$\text{Reliability degree} = \frac{\text{The number of matches}}{\vphantom{x}} \div \left( 10 \times \sqrt{\text{Total number of chemical substances}} \right) \cdots (1)$$

In Expression (1), "the number of matches" means the number of the chemical substances in which the type of the index value and the index value match the type of the index value of the partial structure and the index value extracted by the first search among the chemical substances extracted by the second search. As a specific example, a case will be described in which "partial graph B" is included in the structure data received by the reception unit 40, and "toxicity" and "HIGH" are associated with "partial graph B" in the partial structure data 32. In addition, in this case, a case will be described in which 99 chemical substances including "partial graph B" are included in the past data 34, and the number of the chemical substances of which "toxicity" is "HIGH" is 90 and the number of the chemical substances of which "toxicity" is "LOW" is 9 among the 99 chemical substances.

In this case, the number of matches in Expression (1) is "90", and the total number of the chemical substances is "99". Therefore, in this case, the derivation unit 46 derives the reliability degree of the index value of "partial graph B" as "0.9" as shown in Expression (2).

$$\text{Reliability degree} = 90 \div \left(10 \times \sqrt{99}\right) = 90 \div 99.5 = 0.9 \cdots (2)$$

It should be noted that the method of deriving the reliability degree is not limited to the method using Expression (1). For example, the reliability degree may be derived using an expression other than Expression (1) in which a higher reliability degree is derived as a ratio of the number of matches to the total number of the chemical substances is higher and the number of matches is larger.

The display control unit 48 performs control of displaying the partial structure extracted by the first search and the reliability degree of the partial structure derived by the derivation unit 46 on the display 23. In this case, the display control unit 48 performs control of displaying the reliability degree on the partial structure of the chemical substance, which is the processing target, in a discriminable manner. In addition, in a case in which there are a plurality of partial structures extracted by the first search, the display control unit 48 performs control of displaying the reliability degree in descending order of the reliability degree.

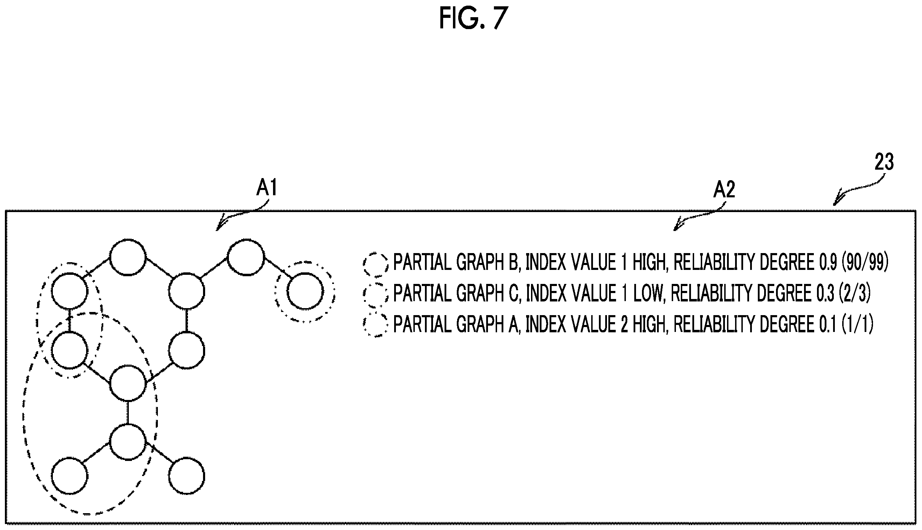

Fig. 7 shows an example of a reliability degree display screen displayed on the display 23 by the control of the display control unit 48. As shown in Fig. 7, the reliability degree display screen according to the present embodiment includes two display regions A1 and A2. In the display region A1, a graph showing the structure of the chemical substance, which is the processing target, is displayed, and the reliability degree of the index value of the partial structure is displayed in a discriminable manner. The partial structure surrounded by a circle in the display region A1 indicates the partial structure extracted by the first search. In addition, the circles in the display region A1 indicate regions filled with colors according to the reliability degree and a light transmittance is set in advance. For example, two threshold values TH1 and TH2 (TH1 > TH2) are set for the reliability degree, and the colors according to the reliability degree are set in advance, such as, red in a case in which the reliability degree of the index value is equal to or greater than the threshold value TH1, yellow in a case in which the reliability degree of the index value is equal to or greater than the threshold value TH2 and smaller than the threshold value TH1, and green in a case in which the reliability degree of the index value is smaller than the threshold value TH2. In the example of Fig. 7, a broken line circle is filled with red, a one-dot chain line circle is filled with yellow, and a two-dot chain line circle is filled with green. As described above, since the colors are different according to the reliability degree of the index value of the partial structure, the reliability degree is discriminable.

In the display region A2, for each of the partial structures extracted by the first search, the type of the index value associated with the partial structure and the index value, the reliability degree of the index value, the number of matches used in a case in which the reliability degree is derived, and the total number of the chemical substances are displayed. In addition, in the display region A2, in a case in which there are the plurality of partial structures extracted by the first search, the reliability degree is displayed in descending order of the reliability degree.

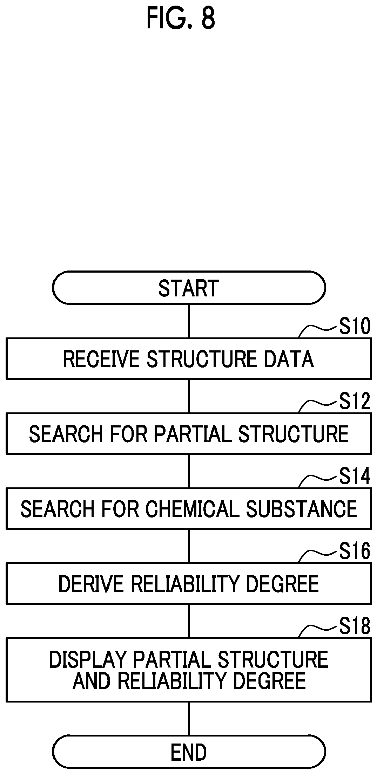

Next, with reference to Fig. 8, the operation of the information processing apparatus 10 according to the present embodiment will be described. The CPU 20 executes the information processing program 30 to execute reliability degree display processing shown in Fig. 8. The reliability degree display processing is executed, for example, in a case in which an execution instruction is input by the user via the input device 24.

In step S10 of Fig. 8, the reception unit 40 receives the structure data indicating the structure of the chemical substance which is the processing target and is input by the user. In step S12, the first search unit 42 performs the first search for searching the structure data received in step S10 for the partial structure included in the partial structure data 32.

In step S14, the second search unit 44 performs the second search for searching the past data 34 for the chemical substance including the partial structure extracted by the first search in step S12. In step S16, as described above, according to Expression (1), the derivation unit 46 derives the reliability degree of the index value of the partial structure extracted by the first search in step S12 based on the total number of the chemical substances extracted by the second search in step S14 and the index value corresponding to the chemical substance.

In step S18, as described above, the display control unit 48 performs control of displaying the partial structure extracted by the first search in step S12 and the reliability degree derived in step S16 for the partial structure on the display 23. The reliability degree display screen shown in Fig. 7 as an example is displayed on the display 23 by the control. In a case in which the processing of step S18 is terminated, the reliability degree display processing is terminated.

By viewing the reliability degree display screen displayed on the display 23, the user can understand, for example, the partial structure having a high risk and high reliability for the high risk among the chemical substances designed by the user himself/herself.

As described above, according to the present embodiment, it is possible to support appropriate screening.

It should be noted that, in the embodiment described above, various processors shown below can be used as the hardware structure of processing units that execute various pieces of processing, such as the reception unit 40, the first search unit 42, the second search unit 44, the derivation unit 46, and the display control unit 48. As described above, the various processors include, in addition to the CPU that is a general-purpose processor that executes software (program) to function as various processing units, a programmable logic device (PLD) that is a processor of which a circuit configuration can be changed after manufacture, such as a field programmable gate array (FPGA), and a dedicated electric circuit that is a processor having a circuit configuration that is designed for exclusive use in order to execute specific processing, such as an application specific integrated circuit (ASIC).

One processing unit may be configured by one of the various processors, or may be configured by a combination of two or more processors of the same type or different types (for example, a combination of a plurality of FPGAs, or a combination of the CPU and the FPGA). Further, a plurality of processing units may be configured by one processor.

A first example of the configuration in which the plurality of processing units are configured by one processor is a form in which one processor is configured by a combination of one or more CPUs and the software and this processor functions as the plurality of processing units, as represented by computers, such as a client and a server. A second example thereof is a form of using a processor that realizes the function of the entire system including the plurality of processing units by one integrated circuit (IC) chip, as represented by a system on chip (SoC) or the like. In this way, the various processing units are configured by using one or more of the various processors described above, as the hardware structure.

Further, more specifically, as the hardware structure of the various processors, an electric circuit (circuitry) in which circuit elements, such as semiconductor elements, are combined can be used.

In addition, in the embodiment described above, an aspect has been described in which the information processing program 30 is stored (installed) in the storage unit 22 in advance, but the present disclosure is not limited to this. The information processing program 30 may be provided in a form of being recorded in a recording medium, such as a compact disc read only memory (CD-ROM), a digital versatile disc read only memory (DVD-ROM), and a universal serial bus (USB) memory. Moreover, the information processing program 30 may be provided in a form being downloaded from an external device via a network.

The disclosure of JP2020-216936 filed on December 25, 2020 is incorporated in the present specification by reference in its entirety. Also, all documents, patent applications, and technical standards described in the present specification are incorporated in the present specification by reference to the same extent as a case in which each document, patent application, and technical standard are specifically and individually described to be incorporated by reference.

**Claims**

1. An information processing apparatus comprising:

   at least one processor,
   wherein the processor
   performs a first search for searching structure data indicating a structure of a chemical substance, which is a processing target, for a partial structure included in partial structure data in which the partial structure of the chemical substance and an index value indicating performance or a structure of the partial structure are associated with each other,
   performs a second search for searching past data in which the structure of the chemical substance and an index value indicating performance or the structure of the chemical substance obtained by an experiment are associated with each other for a chemical substance including the partial structure extracted by the first search,
   derives a reliability degree indicating reliability of the index value of the partial structure extracted by the first search based on a total number of the chemical substances extracted by the second search and an index value corresponding to the chemical substance, and
   performs control of displaying the partial structure extracted by the first search and the reliability degree of the partial structure on a display device.

2. The information processing apparatus according to claim 1,
   wherein the processor performs control of displaying the reliability degree on the partial structure of the chemical substance, which is the processing target, in a discriminable manner.

3. The information processing apparatus according to claim 1 or 2,
   wherein the processor performs control of displaying the reliability degree in descending order of the reliability degree in a case in which there are a plurality of the partial structures extracted by the first search.

4. The information processing apparatus according to any one of claims 1 to 3,
   wherein the processor performs weighting according to the total number of the chemical substances extracted by the second search in a case in which the reliability degree is derived.

5. An information processing method executed by a processor provided in an information processing apparatus, the method comprising:

   performing a first search for searching structure data indicating a structure of a chemical substance, which is a processing target, for a partial structure included in partial structure data in which the partial structure of the chemical substance and an index value indicating performance or a structure of the partial structure are associated with each other;
   performing a second search for searching past data in which the structure of the chemical substance and an index value indicating performance or the structure of the chemical substance obtained by an experiment are associated with each other for a chemical substance including the partial structure extracted by the first search;
   deriving a reliability degree indicating reliability of the index value of the partial structure extracted by the first search based on a total number of the chemical substances extracted by the second search and an index value corresponding to the chemical substance; and
   performing control of displaying the partial structure extracted by the first search and the reliability degree of the partial structure on a display device.

6. An information processing program causing a processor provided in an information processing apparatus to execute a process comprising:

   performing a first search for searching structure data indicating a structure of a chemical substance, which is a processing target, for a partial structure included in partial structure data in which the partial structure of the chemical substance and an index value indicating performance or a structure of the partial structure are associated with each other;
   performing a second search for searching past data in which the structure of the chemical substance and an index value indicating performance or the structure of the chemical substance obtained by an experiment are associated with each other for a chemical substance including the partial structure extracted by the first search;
   deriving a reliability degree indicating reliability of the index value of the partial structure extracted by the first search based on a total number of the chemical substances extracted by the second search and an index value corresponding to the chemical substance; and
   performing control of displaying the partial structure extracted by the first search and the reliability degree of the partial structure on a display device.

## FIG. 1

## FIG. 2

# FIG. 3

CAUTION

LOWER RISK THAN LEFT SIDE

# FIG. 4

32

| PARTIAL STRUCTURE | TYPE | INDEX VALUE |
|---|---|---|
| PARTIAL GRAPH A | INDEX VALUE 2 | HIGH |
| PARTIAL GRAPH B | INDEX VALUE 1 | HIGH |
| PARTIAL GRAPH C | INDEX VALUE 1 | LOW |
| ... | ... | ... |

# FIG. 5

34

| OVERALL STRUCTURE | TYPE | INDEX VALUE |
|---|---|---|
| GRAPH A | INDEX VALUE 1 | HIGH |
| GRAPH B | INDEX VALUE 2 | HIGH |
| ... | ... | ... |

# FIG. 6

INFORMATION PROCESSING APPARATUS 10

| RECEPTION UNIT 40 | FIRST SEARCH UNIT 42 | SECOND SEARCH UNIT 44 | DERIVATION UNIT 46 |
|---|---|---|---|

| | PARTIAL STRUCTURE DATA 32 | PAST DATA 34 | DISPLAY CONTROL UNIT 48 |

# FIG. 7

A1  A2  23

PARTIAL GRAPH B, INDEX VALUE 1 HIGH, RELIABILITY DEGREE 0.9 (90/99)
PARTIAL GRAPH C, INDEX VALUE 1 LOW, RELIABILITY DEGREE 0.3 (2/3)
PARTIAL GRAPH A, INDEX VALUE 2 HIGH, RELIABILITY DEGREE 0.1 (1/1)

EP 4 270 401 A1

# FIG. 8

```
         ┌─────────────────────┐
         │        START        │
         └──────────┬──────────┘
                    │        ⌇S10
    ┌───────────────┴────────────────┐
    │     RECEIVE STRUCTURE DATA      │
    └───────────────┬────────────────┘
                    │        ⌇S12
    ┌───────────────┴────────────────┐
    │    SEARCH FOR PARTIAL STRUCTURE │
    └───────────────┬────────────────┘
                    │        ⌇S14
    ┌───────────────┴────────────────┐
    │  SEARCH FOR CHEMICAL SUBSTANCE  │
    └───────────────┬────────────────┘
                    │        ⌇S16
    ┌───────────────┴────────────────┐
    │     DERIVE RELIABILITY DEGREE   │
    └───────────────┬────────────────┘
                    │        ⌇S18
    ┌───────────────┴────────────────┐
    │    DISPLAY PARTIAL STRUCTURE    │
    │     AND RELIABILITY DEGREE      │
    └───────────────┬────────────────┘
                    │
         ┌──────────┴──────────┐
         │         END         │
         └─────────────────────┘
```

## INTERNATIONAL SEARCH REPORT

| | International application No. |
|---|---|
| | **PCT/JP2021/043214** |

### A. CLASSIFICATION OF SUBJECT MATTER

*G16C 20/64*(2019.01)i; *C07B 61/00*(2006.01)i; *G06F 16/9038*(2019.01)i
FI:  G16C20/64; C07B61/00 Z; G06F16/9038

According to International Patent Classification (IPC) or to both national classification and IPC

### B. FIELDS SEARCHED

Minimum documentation searched (classification system followed by classification symbols)

  G16C20/00-20/90; C07B61/00; G06F16/00-16/958

Documentation searched other than minimum documentation to the extent that such documents are included in the fields searched

   Published examined utility model applications of Japan 1922-1996
   Published unexamined utility model applications of Japan 1971-2022
   Registered utility model specifications of Japan 1996-2022
   Published registered utility model applications of Japan 1994-2022

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | US 2015/0006438 A1 (INTERNATIONAL BUSINESS MACHINES CORPORATION) 01 January 2015 (2015-01-01) | 1-6 |
| A | WO 2009/118845 A1 (FUJITSU LIMITED) 01 October 2009 (2009-10-01) | 1-6 |

☐ Further documents are listed in the continuation of Box C.     ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **12 January 2022** | **25 January 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2021/043214**

| Patent document cited in search report | | | Publication date (day/month/year) | Patent family member(s) | | | Publication date (day/month/year) |
|---|---|---|---|---|---|---|---|
| US | 2015/0006438 | A1 | 01 January 2015 | US | 2015/0006439 | A1 | |
| | | | | US | 2017/0098062 | A1 | |
| | | | | US | 2017/0098063 | A1 | |
| WO | 2009/118845 | A1 | 01 October 2009 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009118845 A1
- JP 2015052990 A
- JP 2020216936 A